# EUROPÄISCHE PATENTSCHRIFT

(11) **EP 2 523 722 B1**
(45) Veröffentlichungstag und Bekanntmachung des Hinweises auf die Patenterteilung: **14.10.2015**
(21) Anmeldenummer: 11700838.3
(22) Anmeldetag: 14.01.2011
(51) Int. Cl.: A61N 1/36, A61F 9/00, A61F 9/02, G02C 11/00, A61N 1/20, A61N 1/04, A61N 1/32

(54) **VORRICHTUNG ZUR ELEKTROSTIMULATION**
ELECTROSTIMULATION DEVICE
APPAREIL D'ÉLECTROSTIMULATION

(30) Priorität: 08.07.2010 DE 102010027201; 15.01.2010 DE 202010001150 U
(43) Veröffentlichungstag der Anmeldung: 21.11.2012
(73) Patentinhaber: Okuvision GmbH, 72770 Reutlingen (DE)
(72) Erfinder: EIPPER, Carmen, 71126 Gäufelden (DE); WROBEL, Walter-G., 72770 Reutlingen (DE)
(74) Vertreter: Witte, Weller & Partner Patentanwälte mbB
(86) Internationale Anmeldenummer: PCT/EP2011/050447
(87) Internationale Veröffentlichungsnummer: WO 2011/086150

(56) Entgegenhaltungen:
- EP-A2- 1 941 829
- WO-A1-2005/068018
- WO-A1-2009/142447
- US-A- 5 154 174
- US-A- 5 360 438
- US-A1- 2004 230 228
- US-A1- 2008 058 793
- FLORIAN GEKELER, ANDRE MESSIAS, MAX OTTINGER, KARL ULRICH BARTZ-SCHMIDT, AND EBERHART ZRENNER: "Phosphenes Electrically Evoked with DTL Electrodes: A Study in Patients with Retinitis Pigmentosa, Glaucoma, and Homonymous Visual Field Loss and Normal Subjects", INVESTIGATIVE OPHTHALMOLOGY & VISUAL SCIENCE, Bd. 47, Nr. 11, 1. November 2006 (2006-11-01), Seiten 4966-4974, XP002640539, DOI: 10.1167

## Beschreibung

Die vorliegende Erfindung betrifft eine Vorrichtung zur Elektrostimulation des Auges, mit einem brillenartigen Gestell, das ein Nasenteil und eine mit dem Nasenteil verbundene Anordnung zum Halten des Gestells an dem Kopf des Patienten aufweist, wobei an dem Nasenteil zumindest eine Stimulationselektrode angeordnet ist.

Eine derartige Vorrichtung ist aus der US 2004/0176820 A1 bekannt.

Die bekannte Vorrichtung sowie weitere Vorrichtungen aus dem Stand der Technik werden eingesetzt, um elektrische Stimulationssignale in das Auge zu leiten, weil sich herausgestellt hat, dass dadurch bestimmte Formen der Makuladegeneration und anderer Augenkrankheiten stabilisiert und sogar verbessert werden können.

Netzhautdegeneration ist eine wesentliche Ursache für Erblindungen in den Industriestaaten. Verschiedene Untersuchungen geben Hinweise darauf, dass durch geringe elektrische Ströme, die durch die Netzhaut fließen, die erbliche, altersbedingte oder plötzlich auftretende Degeneration der Netzhaut verzögert werden kann.

Dies eröffnet die Möglichkeit, durch regelmäßige, also beispielsweise tägliche oder wöchentliche elektrische Stimulation des Auges den allmählichen Verlust des Sehvermögens verzögern zu können, um so betroffenen Patienten länger Teile ihres Augenlichts zu erhalten oder sogar Teile des Augenlichts wiederzugeben.

Diese Behandlung sollte zweckmäßigerweise vom Patienten selbst und zu Hause durchgeführt werden. Dabei muss im Extremfall angenommen werden, dass der Patient praktisch blind ist. Hieraus ergeben sich wesentliche Forderungen an die eingangs erwähnte Vorrichtung.

Die aus der eingangs erwähnten US 2004/0176820 A1 bekannte Vorrichtung ist eine Art Brille mit einem beide Augen nach Art einer Taucherbrille abdeckenden Nasenteil, an dem mehrere Elektroden angeordnet ist, mit denen Kontakt zu dem Auge hergestellt wird, wenn die Brille aufgesetzt wird.

Die bekannte Brille ist dazu mit einer Art Elastikband ausgerüstet, über das die von dem Patienten aufgesetzte Brille nach Art einer Taucherbrille an dem Kopf gehalten wird, so dass sie dicht an dem Gesicht um den Augenbereich herum anliegt.

Um die Augenpartie herum sind mehrere Elektroden vorgesehen, die punktförmig Kontakt zu dem die Augen unmittelbar umgebenden Gewebe herstellen.

Es wird erwähnt, dass auch eine einzelne Elektrode verwendet werden kann, die auf dem geschlossenen Augenlid oder an beliebigem anderem Augengewebe anliegen kann.

In nicht näher bezeichneter Weise werden die Elektroden mit einem Stimulationsgerät verbunden, das elektrische Signale erzeugt, die als Stimulationssignale über die Elektroden in die Augen geführt werden. Die erforderliche Gegenelektrode wird beliebig am Körper des Patienten angebracht.

Der Erfinder der US 2004/0176820 A1 berichtet, dass mit der bekannten Brille bei Anlegen von Stimulationssignalen bestimmter Frequenzen, Stromstärken und Spannungshüben verschiedene Formen der Netzhautdegeneration zum Stillstand gebracht werden können, wobei sich teilweise der Zustand des Patienten sogar wieder verbessert.

Einen ähnlichen Ansatz verfolgt die in der WO 2005/077452 A1 beschriebene Vorrichtung, mit der ebenfalls die Elektrostimulation des Auges möglich ist.

Bei dieser Vorrichtung sind die Elektroden in Form von Schaumstoffkissen ausgebildet, die auf die geschlossenen Augenlider aufgelegt werden. Die Gegenelektrode wird mit der Hand des Patienten verbunden.

Auch hier wird ein Stimulationsgerät an die Elektroden angeschlossen, um elektrische Stimulationssignale in die Augen zu leiten.

Auch die US 5,522,864 A beschreibt die Verwendung einer auf das geschlossene Augenlid aufzulegenden Schaumstoffelektrode für die Elektrostimulation am Auge, wobei die Elektrode und ggf. Teile der Stimulationselektronik über ein Stirnband am Kopf des Patienten befestigt werden.

Die US 6,035,236 A und die US 6, 275,735 B1 beschreiben Verfahren zur Elektrostimulation, bei denen an bestimmte Stimulationspunkte auf der Haut um das Auge herum Stimulationssignale angelegt werden.

Die EP 0 325 201 A2 beschreibt ein Stimulationsgerät zur Verbesserung des Sehvermögens, das ein brillenähnliches Gestell umfasst, an dem ein federndes Element angeordnet ist, das eine Platte gegen das geschlossene Augenlid drückt. Auf der Platte ist eine flächige Elektrode vorgesehen, die auf dem geschlossenen Augenlid aufliegt und Stimulationssignale abgibt.

Das Dokument DE 90 11 254 U beschreibt eine therapeutische Brille, um bestimmte Hautareale um das Auge herum elektrisch zu stimulieren. In die Brille sind Elektrodenplatten eingelassen, die bei aufgesetzter Brille mit den zu stimulierenden Hautbereichen in unmittelbaren Kontakt gelangen.

Bei allen insoweit beschriebenen Vorrichtungen ist von Nachteil, dass die elektrische Stimulation des Auges durch auf die Haut aufgelegte Elektroden erfolgt, wie es auch bereits aus anderen Anwendungsbereichen der Elektrostimulation bekannt ist.

So wurde die Elektrostimulation allgemein bereits seit dem 18. Jahrhundert zur Behandlung unterschiedlichster Krankheitsbilder vorgeschlagen, darunter auch für Augenerkrankungen. Dabei wurden unterschiedlichste Gestaltungen für die verwendeten Elektroden vorgeschlagen.

Eine Gestaltungsmöglichkeit besteht darin, mit den geöffneten Augen in ein Wasserbad aus physiologischer Kochsalzlösung zu schauen, wobei das Wasserbad Elektroden enthält, die mit dem Stimulationsgerät verbunden sind. Dieses Verfahren wird Iontophorese genannt. Wegen seiner Umständlichkeit hat die Iontophorese jedoch keine weite Verbreitung gefunden.

An die Augen angelegte Elektroden finden auch Verwendung in der Elektroretinographie (ERG). Bei diesem ophthalmologischen Diagnoseverfahren werden Elektroden in Augennähe angebracht, Lichtblitze in das Auge eingeleitet und an den Elektroden die entsprechenden Nervenströme des optischen Nervs gemessen.

Im Rahmen der Anwendung der ERG hat sich ergeben, dass bei der Verwendung von auf der Haut aufliegenden Elektroden die Signalamplitude stark schwankt und von dem aktuellen Zustand der Haut abhängt, also von ihrem Fettgehalt, ihrer Feuchtigkeit sowie beispielsweise der Tatsache, ob sie frisch gewaschen wurde.

Diese Effekte sind für die ERG nur in geringem Maß störend, da die Elektroden hier zur Ableitung von Messströmen verwendet werden, wo der Zeitverlauf der Signale von Interesse ist.

Für die im Rahmen der vorliegenden Anmeldung geplante therapeutische Anwendung sind diese Effekte jedoch nicht akzeptabel. Wenn der Hautwiderstand nämlich von Applikation zu Applikation sehr unterschiedlich ist, kann nicht gewährleistet werden, dass die an der Retina erzeugte elektrische Feldstärke reproduzierbar ist und ein Therapieerfolg erwartet werden kann.

Auch in der ERG werden zur Vermeidung dieser Effekte vorzugsweise Elektroden verwendet, die einen direkten Kontakt mit feuchten Körperoberflächen herstellen, der niederohmig ist. In der Augenheilkunde wird daher in der Regel mit der Stimulationselektrode die Cornea, also die Augenoberfläche, kontaktiert. Auf der Haut wird dann eine großflächige Gegenelektrode angebracht, deren große Fläche einen geringen Übergangswiderstand und damit reproduzierbare Verhältnisse gewährleistet.

Zu den aus der ERG bekannten Augenelektroden zählen zum einen metallisch beschichtete Kontaktlinsen, wie sie in der US 7,020,527 für die Elektrostimulation verwendet werden. Diese Kontaktlinsen gelten als schwer verträglich und schmerzhaft, da aus Kostengründen ihre Krümmung nicht präzise an das Auge angepasst wird.

Aus der US 5,154,174 A ist es bekannt, gebogene Drahtschlingen als Elektroden für die ERG zu verwenden, die in die Bindesackhaut des Auges eingelegt werden. Diese auch als Hawlina-Elektroden bekannten Elektroden sind deutlich verträglicher als die metallisch beschichteten Kontaktlinsen, aber für blinde Patienten nur schwer zu handhaben, weil sie die genaue Form und den Sitz der Drahtschlinge nicht kontrollieren können. Die Fixierung am Kopf erfolgt in der Regel über Heftpflaster, was den Nachteil aufweist, dass sich die Position der Elektrode über der Gesamtzeit der Stimulation verändern kann.

Weite Verbreitung in der ERG haben auch so genannte DTL-Elektroden gefunden, wie sie von der Firma Diagnosys LLC, Lowell, MA, USA, angeboten werden.

Diese DTL-Elektroden bestehen aus einem feinen Draht, der so auf die Cornea aufgelegt wird, dass er an der Kante Augenlid/Cornea aufliegt und guten Kontakt zur Cornea aufweist. Links und rechts vom behandelten Auge wird der Draht mit Pflastern befestigt.

Diese Elektroden sind in der ERG vielfach getestet, sie weisen von den bekannten ERG-Elektroden die beste Verträglichkeit auf, haben aber den Nachteil, dass zu ihrer Applikation in der klinischen Praxis eine erhebliche Fingerfertigkeit von dem Arzt oder Krankenpfleger gefordert wird. Diese Fähigkeit ist bei einem Patienten in der Regel nicht vorhanden, erst recht nicht bei einem erblindeten Patienten. Diese Elektroden können Patienten daher nicht selbständig anwenden.

Die US 5,360,438 beschreibt eine Brille, an deren Nasenauflagen zwei punktförmige Elektroden vorgesehen sind, die der Stimulation von Kranialnerven dienen.

Die EP 1 941 829 A2 zeigt in Fig. 30 eine Brille, an der eine Empfangseinheit vorgesehen ist, die mit einer Kontakteinheit in Funkverbindung ist, die der Neurostimulation dienen kann und auf dem Auge befestigt wird.

Gekeler et al., "Phosphenes Electrically Evoked with DTL elektrodes: A Study in Patients with Retinitis Pigmentosa, Glaucoma, and Homonymus Visual Field Loss and Normal Subjects", in Investigative Ophthalmology & Visual Science, 2006; 47; 4966-4974 beschreiben die Verwendung von DTL-Elektroden in einer Studie an Patienten mit Retinadegeneration. In dieser Studie wurde gezeigt, dass die gepulste elektrische Stimulation der Retina oberhalb einer bestimmten Intensität zu der Wahrnehmung von elektrisch ausgelösten Lichtphänomenen, sogenannten Phosphenen führt.

Vor diesem Hintergrund liegt der vorliegenden Erfindung die Aufgabe zugrunde, die eingangs erwähnte Vorrichtung derart weiterzubilden, dass sie einen guten, reproduzierbaren Kontakt zwischen der Stimulationselektrode und dem zu behandelnden Auge herstellt, gleichzeitig aber auch von einem blinden oder nahezu blinden Patienten problemlos zu Hause benutzt werden kann.

Diese Aufgabe wird bei der eingangs erwähnten Vorrichtung dadurch gelöst, dass an dem Nasenteil zumindest zwei Elektrodenhalter vorgesehen sind, zwischen denen eine auswechselbare, drahtförmige Stimulationselektrode eingespannt ist, die sich beim Aufsetzen der Vorrichtung an die Cornea anlegt.

Die der Erfindung zugrunde liegende Aufgabe wird auf diese Weise vollständig gelöst.

Der Erfinder der vorliegenden Anmeldung hat nämlich erkannt, dass es möglich ist, die Art der Positionierung sowie die gute Verträglichkeit und der gute sowie reproduzierbare elektrische Kontakt einer drahtförmigen Elektrode, insbesondere einer DTL-Elektrode, mit einer Vorrichtung zur stabilen und präzisen Positionierung am Kopf bzw. Auge des Patienten zu verbinden.

Diese Vorrichtung wird einmalig von einem Fachmann genau auf den jeweiligen Patienten eingestellt, der dann nur noch eine vorgefertigte Drahtelektrode in die Elektrodenhalter einhängen und die Vorrichtung aufsetzen und an ein Stimulationsgerät anschließen muss.

Danach ist die Vorrichtung in bekannter Weise mit dem Stimulationsgerät zu verbinden, woraufhin die Behandlung beginnen kann.

Die Art des verwendeten Stimulationsgerätes und die Signalform der verwendeten Stimulationssignale ist nicht Gegenstand der vorliegenden Erfindung, sie müssen nur für die Elektrostimulation der Retina geeignet sein. Es können somit beispielsweise die Stimulationsgeräte und Signalformen zum Einsatz kommen, die in den eingangs erwähnten Dokumenten US 2004/0176820 A1, WO 2005/077452 A1, US 5,522,864 A, US 6,035,236 A und US 6, 275,735 B1 beschrieben sind.

Als Stimulationselektrode können dabei beliebige Drahtelektroden, vorzugsweise jedoch DTL-Elektroden verwendet werden, wie sie beispielweise von der eingangs erwähnten Firma Diagnosys LLC bezogen werden können. Die Stimulationselektrode ist vorzugsweise aus einem Edelmetall, einer Edelstahllegierung oder einem metallisch beschichteten Kunststofffaden gefertigt. Bewährt hat sich beispielsweise ein feiner, silberbeschichteter Nylon- also Polyamid-Faden.

Die Stimulationselektroden sind austauschbar, so dass verschmutzte oder defekte Stimulationselektroden durch neue ersetzt werden können, ohne eine neue Vorrichtung beschafft und angepasst werden muss. Dies ist vor allem unter Kostengesichtspunkten von Vorteil.

Dabei ist es bevorzugt, wenn die Stimulationselektrode elektrisch leitend mit zumindest einem der Elektrodenhalter verbunden ist, wobei weiter vorzugsweise jeder Elektrodenhalter an seinem freien Ende mit einer Aufnahme für die Stimulationselektrode versehen ist.

Hier ist von Vorteil, dass die empfindliche Stimulationselektrode nur beim Austauschen befestigt und gleichzeitig kontaktiert wird, danach erfolgt der elektrische Anschluss an das Stimulationsgerät über die Elektrodenhalter, an die beispielsweise immer dann ein Anschlusskabel angeklemmt wird, wenn die Vorrichtung zum Einsatz kommt.

Es ist jedoch bevorzugt, wenn mit zumindest einem der Elektrodenhalter ein Kabel verbunden ist. Das Kabel ist entweder als Anschlusskabel zum unmittelbaren Anschluss an ein Stimulationsgerät ausgebildet oder mit einer an der Vorrichtung angeordneten Anschlusslasche verbunden.

Diese Maßnahme erhöht den Bedienungskomfort und die Betriebssicherheit. Der Patient muss nach dem Aufsetzen der Vorrichtung nur noch die an den Elektrodenhaltern bereits vorhandenen Anschlusskabel mit dem Stimulationsgerät verbinden.

Wenn an der Vorrichtung Anschlusslaschen für den Anschluss der Anschlusskabel vorgesehen sind, lässt sich die Vorrichtung nach dem Gebrauch leichter verstauen, wobei die Elektrodenhalter auch besser geschützt sind, da die Anschlusskabel vorher abgenommen werden können. Ferner findet im Einsatz eine Art Zugentlastung statt, denn die Anschlusskabel "ziehen" nicht unmittelbar an den Elektrodenhaltern.

Allgemein ist es bevorzugt, wenn die Elektrodenhalter mit einem elastisch nachgiebigen, langgestreckten Körper ausgebildet sind, der vorzugsweise als Spiralfeder ausgebildet und/oder aus biegsamem Kunststoff gefertigt ist, wobei vorzugsweise jeder Elektrodenhalter eine Halterung aufweist, über die er verstellbar und austauschbar an dem Nasenteil angeordnet ist.

Hier ist von Vorteil, dass sich die Elektrodenhalter beim Aufsetzen der Vorrichtung und damit verbundenem Anlegen des Stimulationselektrode an die Cornea unter dem Zug der sich auswölbenden Stimulationselektrode so verbiegen, dass sie einerseits die Stimulationselektrode straff gespannt halten, andererseits aber keine zu großen Druck auf die Cornea ausüben.

Für verschiedene Patienten können ferner unterschiedliche Elektrodenhalter erforderlich sein, was sowohl ihre Länge als auch ihre Flexibilität und Einstellbarkeit angeht. Daher werden für die nur in begrenzter Anzahl erforderlichen Gestelle unterschiedliche Elektrodenhalter vorrätig gehalten, die austauschbar sind.

Gemäß einem weiteren Ausführungsbeispiel sind die Elektrodenhalter als leitfähige Stifte, vorzugsweise aus V2A-Stahl, ausgebildet, wobei vorzugsweise die zumindest eine Stimulationselektrode mit einem der Stift fest verbunden ist und an dem anderen Stift durch eine Öse geführt und auf Zug belastet ist, vorzugsweise durch einen Gewichtskörper.

Hier ist von Vorteil, dass einfach aufgebaute und leicht einzustellende Elektrodenhalter verwendet werden, die jedoch so stabil sind, dass sie sich beim Auswechseln der Stimulationselektroden nicht verbiegen oder verstellen.

Ferner wird durch die Verwendung von Gewichtskörpern das Anlegen neu eingesetzter Stimulationselektroden an das Auge derart vereinfacht, dass dies auch von einem stark sehbehinderten Patient selbst vorgenommen werden kann.

Dabei kann die zumindest eine Stimulationselektrode auch als mit einem Gewichtskörper verbundene Schlaufe ausgebildet sein, die vor der Benutzung der erfindungsgemäßen Vorrichtung lediglich in beide Elektrodenhalter so eingehängt wird, dass sich die Stimulationselektrode zwischen den beiden Elektrodenhaltern erstreckt und durch den Gewichtskörper gespannt wird, der unterhalb und zwischen den Elektrodenhaltern hängt.

Die Elektrodenhalter sind dazu als Stifte aus einem elektrisch leitfähigen Material ausgebildet, die an ihrem inneren, dem Patienten zugewandten freien Ende jeweils eine nach oben offene Nut aufweisen, in die die Stimulationselektrode auch von einem stark sehbehinderten oder gar blinden Patienten eingehängt werden kann.

Um das Einhängen oder Einlegen der Stimulationselektrode weiter zu erleichtern, kann der Stift jeweils mit zumindest einer schräg auf die Nut zu verlaufenden Fläche versehen sein, auf der die aufgelegte Stimulationselektrode in die Nut hineinrutscht.

Der in seiner Längsrichtung verstellbare Elektrodenhalter kann dabei höhenverstellbar an der Vorrichtung befestigt sein, wozu vorzugsweise ein weiterer Stift vorgesehen ist, der an seinem einen Ende höhenverstellbar an der Vorrichtung angeordnet ist und an seinem anderen Ende einen weiteren Stift trägt, der quer zu dem weiteren Stift verstellbar ist und an seinem freien, dem Patienten zugewandten Ende zur Aufnahme der Stimulationselektrode ausgebildet ist.

Das Gestell und die Elektrodenhalter können dann einmalig von einer normalsichtigen Person an die Kopf- und Gesichtsform des Patienten angepasst werden. Danach können auch praktisch blinde Patienten die Stimulationselektroden in die Elektrodenhalter einhängen und das Gestell präzise und reproduzierbar aufsetzen. Dabei legen sich die Stimulationselektroden so an das zu behandelnde Auge an, dass sie auf der Cornea und zwischen den Lidrändern zu liegen kommen.

Die Stimulationselektroden sind also austauschbar und als Verbrauchsmaterial anzusehen.

Vor diesem Hintergrund betrifft die vorliegende Erfindung auch eine Stimulationselektrode zur Verwendung mit der neuen Vorrichtung, die als Schlaufe aus einem metallisch beschichteten Kunststofffaden ausgebildet und mit einem Gewichtskörper verbunden ist.

Schließlich ist es bevorzugt, wenn das Nasenteil einen Nasenbügel und zumindest einen Rahmen oder Augenbügel aufweist, an dem die beiden Elektrodenhalter angeordnet sind, wobei die Anordnung zum Halten des Gestells zwei an dem Nasenteil befestigte Seitenbügel aufweist, die in sich flexibel und/oder klappbar an dem Nasenteil angelenkt sind.

Der Nasenbügel kann dabei gegenüber den Rahmen oder Augenbügeln verstellbar angeordnet sein, vorzugsweise über einen Haltestift an einem Nasenhalter verstellbar sein, an dem zwei Augenbügel festgelegt sind, von denen zumindest einer mit zwei Stifthaltern versehen sind, die verschiebbar auf dem Augenbügel sitzen und jeder einen höhenverstellbaren Haltestift tragen, an denen jeweils ein in seiner Längsrichtung verstellbarer Elektrodenhalter gehalten ist.

An jedem Elektrodenhalter ist an dessen freiem, dem Patienten zugewandten Ende eine Aufnahme für die Stimulationselektrode vorgesehen, vorzugsweise eine nach oben offene Nut. Die gewählte Ausgestaltung ermöglicht es dabei, dass das freie Ende mit der Aufnahme in den drei Raumrichtungen gegenüber dem Augenbügel verstellt werden kann.

Hier ist von Vorteil, dass sich das Gestell nach Art einer Brille gut an die Kopfform des Patienten anpassen lässt, und dass dennoch eine gute Justierung und Ausrichtung der Elektrodenhalter zu den Augen des Patienten möglich ist.

Wenn bei den insoweit beschriebenen Vorrichtungen zwei Stimulationselektroden verwendet werden, kann der Stromkreis über diese beiden Stimulationselektroden geschlossen werden, die folglich in Serie geschaltet sind und beide über ein eigenes Anschlusskabel mit dem Stimulationsgerät verbunden werden.

Wird nur eine Stimulationselektrode verwendet, oder werden die beiden Stimulationselektroden elektrisch parallel geschaltet, so ist eine Gegenelektrode vorgesehen, die vorzugsweise im Schläfenbereich des Patienten angelegt und über ein an der Vorrichtung festgelegtes Anschlusskabel mit dem Stimulationsgerät verbunden wird

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische perspektivische Darstellung der neuen Vorrichtung, mit der beide Augen behandelt werden;
- Fig. 2: eine Draufsicht auf die Vorrichtung aus Fig. 1;
- Fig. 3: eine alternative Ausgestaltung der Vorrichtung aus Fig. 1;
- Fig. 4: einen Elektrodenhalter, der bei den Vorrichtungen aus den Fig. 1 bis 3 verwendet wird;
- Fig. 5: eine skizzenhafte perspektivische und ausschnittsweise Darstellung einer nicht zur Erfindung zählenden Gesichtsmaske, an der eine Stimulationselektrode angeordnet und über Druckknöpfe gehalten ist;
- Fig. 6: einen Schnitt durch einen der Druckknöpfe mit Anschlusskabel und Stimulationsselektrode;
- Fig. 7: eine weitere alternative Ausgestaltung der Vorrichtung aus Fig. 1;
- Fig. 8: eine alternative Ausgestaltung der Vorrichtung aus Fig. 7 in Draufsicht und Ansicht vom Patienten aus;
- Fig. 9: eine in zwei Elektrodenhalter eingehängte und mit einem Gewichtskörper beschwerte Stimulationselektroden-Schlaufe; und
- Fig. 10: eine schematische Seitenansicht eines inneren freien Endes eines Elektrodenhalters mit einer Aufnahme für eine Stimulationselektrode.

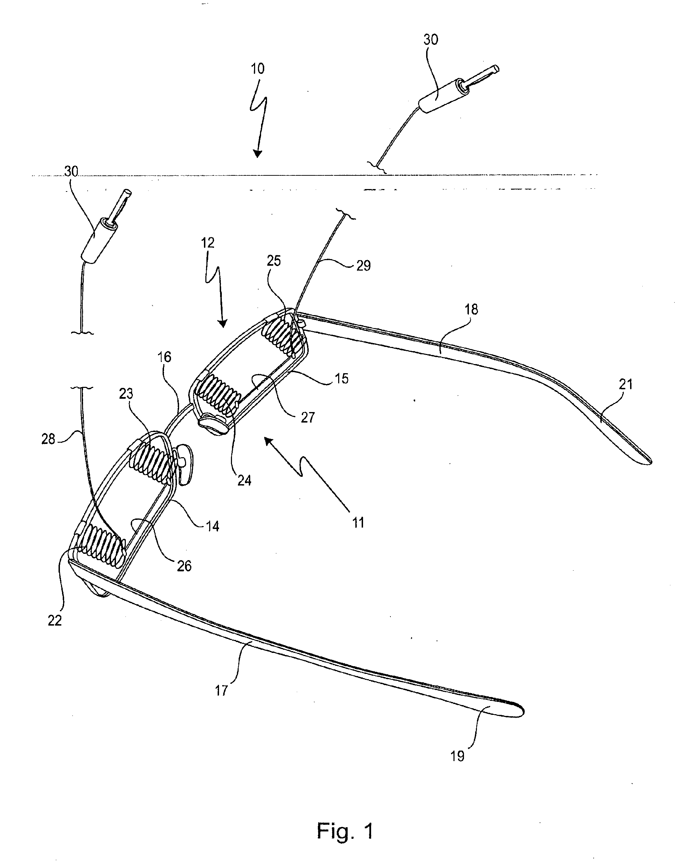

In Fig. 1 ist mit 10 eine Vorrichtung gezeigt, die zur Elektrostimulation des Auges dient. Die Vorrichtung 10 umfasst ein brillenartiges Gestell 11, das ein Nasenteil 12 aufweist, an dem zwei Rahmen 14 und 15 ausgebildet sind, die der Lage des Auges des Patienten entsprechen. Die beiden Rahmen 14 und 15 sind über einen Nasenbügel 16 miteinander verbunden.

Wie bei einer Brille üblich, ist das Nasenteil 12 mit einer Anordnung versehen, um das Gestell 11 am Kopf eines Patienten zu halten. Diese Anordnung weist zwei Seitenbügel 17 und 18 auf, die klappbar an dem Nasenteil 12 angelenkt und an ihrem freien Ende 19 und 21 abgebogen ausgebildet sind, so dass das Gestell 11 an die Physiognomie eines Patienten optimal angepasst werden kann.

An jedem Rahmen 14 und 15 sind zwei elastisch nachgiebige Elektrodenhalter 22 und 23 bzw. 24 und 25 vorgesehen, die in Richtung der Seitenbügel 17 und 18 sowie quer dazu federnd ausgebildet sind.

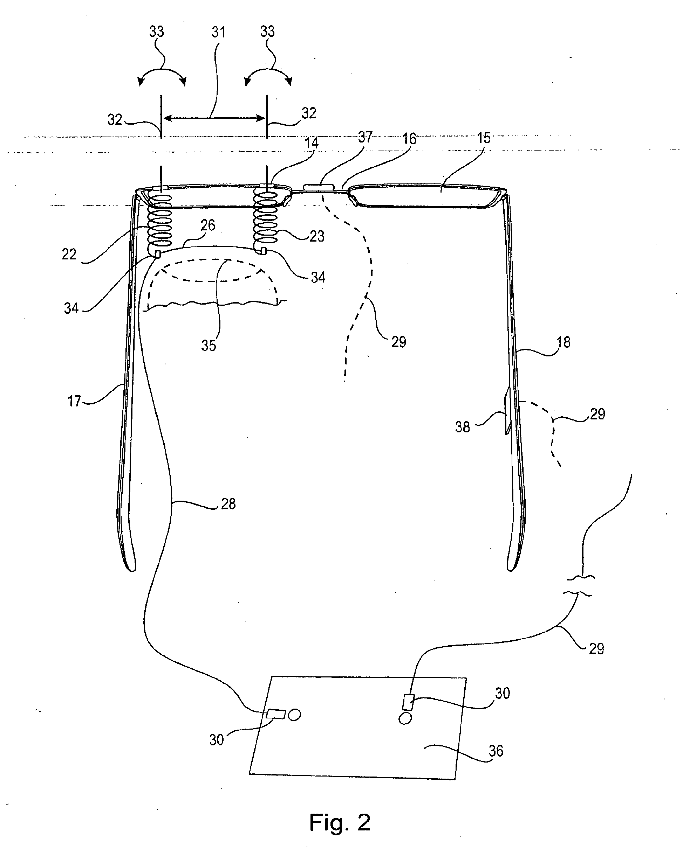

Die Elektrodenhalter 22, 23, 24, 25 sind so ausgebildet, dass zwischen ihnen je eine drahtförmige Stimulationselektrode 26 bzw. 27 eingespannt werden kann, die elektrisch leitend mit dem jeweils äußeren Elektrodenhalter 22 bzw. 25 verbunden ist. Der Elektrodenhalter 22 sowie der Elektrodenhalter 25 sind jeweils mit einem Anschlusskabel 28 bzw. 29 verbunden, das an seinem freien Ende je einen Stecker 30 aufweist, mit dem die Elektrodenhalter 22 und 25 und somit die drahtförmigen Stimulationselektroden 26 und 27 mit den beiden Polen eines Stimulationsgerätes verbunden werden können, wie es weiter unten im Zusammenhang mit Fig. 2 beschrieben wird.

Die Elektrodenhalter 22, 23, 24, 25 sind so ausgebildet, dass sie in Längsrichtung federn, sich aber auch seitlich verschieben lassen. Im einfachsten Fall sind die Elektrodenhalter reine Spiralfedern, die geeignet an den Rahmen 14 und 15 befestigt sind.

Wenn das brillenartige Gestell 11 geeignet an die Physiognomie des Patienten angepasst ist, liegen die Elektrodenhalter 22, 23, 24, 25 beim Aufsetzen der Vorrichtung 10 auf Höhe des Auges sowie links und rechts davon und ragen so vor, dass sie die Lidecken fast berühren.

Um eine Verletzungsgefahr zu vermeiden, sind die vorstehenden Enden der Elektrodenhalter 22, 23, 24 25 dort abgerundet ausgeführt, wo sie die Stimulationselektrode 26 bzw. 27 tragen.

Die Elektrodenhalter 22, 23, 24, 25 können auf die Augenposition einjustiert werden, wozu sie über in den Fig. nicht gezeigte verschiebbare Muffen und Fixierschrauben an den Rahmen 14 und 15 befestigt sind. Dabei ist sowohl eine Justierung des Abstandes der Elektrodenhalter 22, 23 bzw. 24, 25, also eine Verstellung aufeinander zu und voneinander weg als auch eine Verstellung quer zu ihrem Abstand und ein Verkippen in jede Richtung um ihre Längsachse möglich. Der Abstand und die Längsachsen sind in der Draufsicht der Fig. 2 bei 31 bzw. 32, die Kippbewegung in einer Ebene durch Pfeile 33 dargestellt.

Um eine optimale Anpassung an den individuellen Patienten zu möglichen, sind die Elektrodenhalter 22, 23, 24, 25 austauschbar an den Rahmen 14, 15 angeordnet, so dass je nach Bedarf längere oder kürzere Elektrodenhalter 22, 23, 24, 25 verwendet werden können.

Die Elektrodenhalter 22, 23, 24, 25 enthalten an ihrem freien, zum Auge gelegenen Ende geeignete Aufnahmen, in die der Patient die Stimulationselektroden 26 bzw. 27 einlegen kann. Eine Sicherung der drahtförmigen Stimulationselektroden 26, 27 kann beispielsweise durch einen Klickverschluss oder eine sonst geeignete Rastung erfolgen. In Fig. 2 sind diese Aufnahmen für die beiden Elektrodenhalter 22 und 23 bei 34 angedeutet.

Die Elektrodenhalter 22 und 23 bzw. 24 und 25 können leicht nach außen geneigt sein, so dass sie die Stimulationselektrode 26 bzw. 27 zwischen sich stramm spannen. Diese Spannung der Stimulationselektrode 26, 27 kann auch durch das Anschlusskabel 28, 29 erreicht werden, das von der Stimulationselektrode 26, 27 zu dem Stimulationsgerät führt.

In der Draufsicht der Fig. 2 ist die Situation gezeigt, wenn die Vorrichtung 10 vom Patienten aufgesetzt wurde, so dass die drahtförmigen Stimulationselektrode 26 an der bei 35 angedeuteten Cornea des Auges anliegen. Die Stimulationselektrode 26 wölbt sich dabei so aus, dass sie passend an der Wölbung der Cornea 35 anliegt. Dies ist möglich, weil die Elektrodenhalter 22, 23 so flexibel ausgebildet sind, dass sie beim Anlegen der Stimulationselektrode 26, 27 an die Cornea 35 leicht nach innen gezogen werden können.

Es sei noch bemerkt, dass in Fig. 2 aus Gründen der Übersichtlichkeit nur für den Rahmen 14 die Stimulationselektrode 26 mit den Elektrodenhaltern 22 und 23 gezeigt ist. Der Rahmen 15 kann genauso ausgebildet sein, wobei es auch möglich ist, nur einen der beiden Rahmen 14, 15 mit einer Stimulationselektrode 26 oder 27 zu versehen, wenn beispielsweise nur ein Auges des Patienten behandelt werden muss oder soll.

Bei korrekter Positionierung der Vorrichtung auf Nase und Ohren des Patienten berührt die Stimulationselektrode 26, 27 beim Aufsetzen zunächst die Cornea 35 an ihrer vordersten Stelle. Beim weiteren Aufsetzen der Vorrichtung legt sich die Stimulationselektrode 26, 27 dann an die Cornea 35 an, da die Elektrodenhalter 22, 23, 24, 25 hinreichend nachgiebig ausgebildet sind.

Statt der in den Fig. 1 bis 3 gezeigten Spiralfedern können die Elektrodenhalter 22, 23, 24, 25 auch aus elastisch biegsamem Kunststoffmaterial gefertigt sein.

Die Stimulationselektroden selbst können aus verschiedenen Materialien bestehen. In Frage kommen die Edelmetalle (Pt, Pd, Ir, Ag, Au etc.), aber auch Edelstahllegierungen. Auch mit Edelmetallen beschichtete Kunststofffäden sind geeignet, sie werden beispielsweise bei DTL-Elektroden eingesetzt. Wegen der weiteren Ausgestaltung der Elektroden wird beispielsweise verwiesen auf Hawlina und Konek: New noncorneal HK-loop electrode for clinical electroretinography", Documenta Ophthalmologica 81: 253-259, 1992, und die dort angeführten Zitate.

Nach dem Aufsetzen des Gestells 11 und der Anpassung der Stimulationselektrode 26, 27 an die Cornea 35 werden die Anschlusskabel 28, 29 mit einem Stimulationsgerät 36 verbunden.

Danach schließt der Patient für die Dauer der nachfolgenden Behandlung das zu behandelnde Auge, so dass die Stimulationselektroden 26, 27 auf der Cornea 35 und zwischen den Lidrändern liegen. Auf diese Weise sind die Stimulationselektroden 26, 27 von allen Seiten von feuchtem, also elektrisch gut kontaktiertem Gewebe umgeben.

Wegen der konkreten Ausgestaltungen des Stimulationsgerätes 36 und der Signalformen der Stimulationssignale darf auf die eingangs diskutierten Dokumente verwiesen werden, wobei im Stand der Technik auch andere Stimulationsgeräte und Signalformen für Stimulationssignale bekannt sind, mit denen beispielsweise die Retina eines Patienten stimuliert werden kann.

Bei der Vorrichtung 10 aus Fig. 1 wird jedes Auge von einer Stimulationselektrode 26, 27 kontaktiert, wobei die jeweils andere Elektrode 27, 26 als Gegenelektrode wirkt, der Stromkreis wird sozusagen über die beiden Augen des Patienten geschlossen. Mit anderen Worten, es ist keine zusätzliche Masse- oder Gegenelektrode erforderlich, was die Benutzung für den Patienten sehr einfach macht.

Die Verwendung der jeweils anderen Stimulationselektrode 27, 26 als Gegenelektrode ist auch deshalb möglich, weil bei der elektrischen Stimulation entweder Wechselspannungen oder so genannte biphasische Pulse verwendet werden.

Wenn die Vorrichtung 10 jedoch dazu ausgerichtet, nur ein Auge zu stimulieren, wird entweder an dem Nasenbügel 16 oder an einem der Seitenbügel 17, 18 eine Gegenelektrode 38 angeordnet, von der das zweite Anschlusskabel 29 ausgeht, wie es in Fig. 2 gezeigt ist.

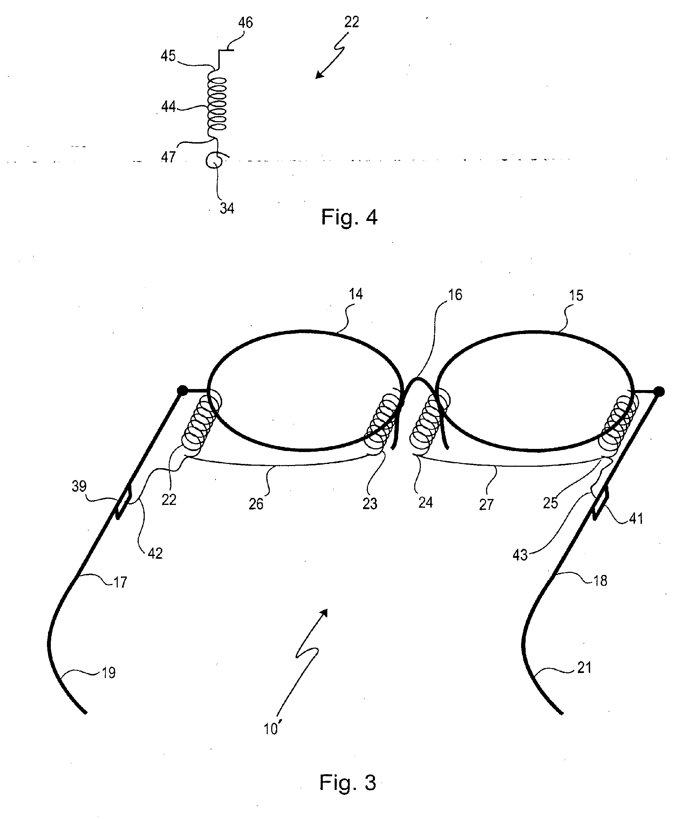

In der Fig. 3 ist noch ein weiteres Ausführungsbeispiel der neuen Vorrichtung 10' gezeigt, bei dem die Rahmen 14 und 15 oval an die Form des Auges angepasst sind, was eine besonders einfache Justierung der Elektrodenhalter 22, 23, 24, 25 ermöglicht.

In Fig. 3 sind dieselben Bezugszeichen wie in Fig. 1 verwendet, so dass wegen der weiteren Einzelheiten auf die obige Beschreibung verwiesen wird.

Bei der Vorrichtung 10' sind an den Seitenbügeln 17, 18 Anschlusslaschen 39, 41 vorgesehen, mit denen die Stimulationselektroden 26, 27 über Kabel 42, 43 verbunden sind. An die Anschlusslaschen 39, 41 werden dann die Anschlusskabel 28, 29 angeschlossen, beispielsweise angeklemmt oder eingesteckt.

Im Gegensatz zu der Vorrichtung 10 aus Fig. 1 sind bei der Vorrichtung 10' die Seitenbügel 17, 18 nicht klappbar an den Rahmen 14, 15 angelenkt sondern in sich flexibel, so dass das Gestell 11 bequem aufgesetzt werden kann und am Kopf hält. Die Anpassung an den Kopf des Patienten erfolgt auch hier über die gebogenen Enden 19, 21, die ggf. nach Erhitzen so verbogen und gekrümmt werden können, dass das Gestell 11 an die Physiognomie eines Patienten optimal angepasst wird.

Wie bereits erwähnt, können die Elektrodenhalter 22, 23, 24, 25 ausgetauscht werden, um einerseits defekte Elektrodenhalter 22, 23, 24, 25 ersetzen zu können, andererseits aber auch die für die Gesichtsform des Patienten passenden Elektrodenhalter 22, 23, 24, 25 an ein und demselben Gestell 11 montieren zu können.

Ein solcher Elektrodenhalter 22 ist beispielhaft in Fig. 4 gezeigt. Er weist einen langgestreckten, elastisch nachgiebigen Körper 44 auf, der an seinem einen Ende 45 eine Halterung 46 trägt über die er an dem Rahmen 14 oder 15 befestigt werden kann. Hier ist eine Schraubverbindung möglich.

An seinem anderen Ende 47 trägt der Elektrodenhalter 22 die bereits erwähnte Aufnahme 34, an der die Stimulationselektrode 26, 27 befestigt wird.

Der Körper 44 ist als Spiralfeder ausgebildet, die aus biegsamem Kunststoff gefertigt ist. Der Körper 44 kann auch als Federzunge ausgebildet sein.

Alternativ zur Ausbildung des Gestells 11 als Brille mit Rahmen 14, 15, Nasenbügel 16 und Seitenbügeln 17, 18 kann das brillenartige Gestell 11 auch als Gesichtsmaske ausgebildet sein.

Unter Gesichtsmaske wird ein maskenartiges Gebilde nach Art einer Larve verstanden, wie es heutzutage verwendet wird, um z.B. nach Nasenbeinbrüchen die Nase zu stabilisieren und gegen mechanische Einwirkungen zu schützen.

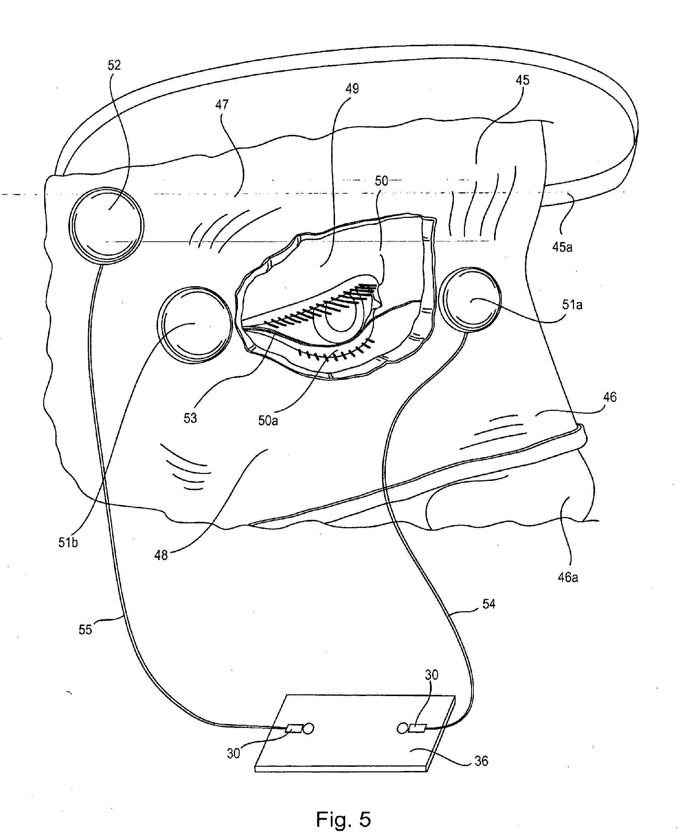

In Fig. 5 ist schematisch und ausschnittsweise eine derartige Gesichtsmaske 45 gezeigt, die ein Nasenteil 46 aufweist, das integral mit der Gesichtsmaske 45 ausgebildet ist. Die Gesichtsmaske 45 wird über ein Elastikband 45a am Kopf des Patienten gehalten.

Die Gesichtsmaske 45 liegt mit dem Nasenteil 46 flächig an einer Nase 46a des Patienten an, wobei ein Stirnteil 47 sowie ein Wangenteil 48 flächig an Stirn bzw. Wange und Schläfe des Patienten anliegen.

In dem Nasenteil 46 ist zumindest eine Augenöffnung 49 vorgesehen, die bei aufgesetzter Gesichtsmaske 45 das Auge 50 des Patienten frei lässt. Selbstverständlich kann die Gesichtsmaske 45 auch zwei Augenöffnungen 49 aufweisen.

Die Gesichtsmaske 45 besteht aus einem thermoplastischen Kunststoff und wird bspw. in einem Sanitätshaus passgenau an die Kopfform des Patienten angepasst, wie dies bspw. bei Sportlern zum Schutz der Nase nach einem Nasenbeinbruch eingesetzt wird.

Nasenseitig neben der Augenöffnung 49 ist ein erster Druckknopf 51a an der Gesichtsmaske 45 befestigt, während an der anderen Seite der Augenöffnung 49, also schläfenseitig, ein zweiter Druckknopf 51b vorgesehen ist. Ein dritter Druckknopf 52 ist im Schläfenbereich der Gesichtsmaske 45, also an dem Wangenteil 47 angeordnet.

Zwischen den Druckknöpfen 51a und 51b erstreckt sich eine Stimulationselektrode 53, die innerhalb der Gesichtsmaske 45, also sozusagen unterhalb der Augenöffnung 49 durch diese hindurch unten am Augenlid 50a des Patienten vorbeiläuft, so dass die Druckknöpfe 51, 52 als Elektrodenhalter wirken.

Der Druckknopf 51 ist mit einem Anschlusskabel 54 verbunden, das zu dem bereits im Zusammenhang mit Fig. 2 beschriebenen Stimulationsgerät 36 führt, mit dem es über einen Stecker 30 verbunden ist, wie dies im Zusammenhang mit den Figuren 1 und 2 auch bereits erläutert wurde.

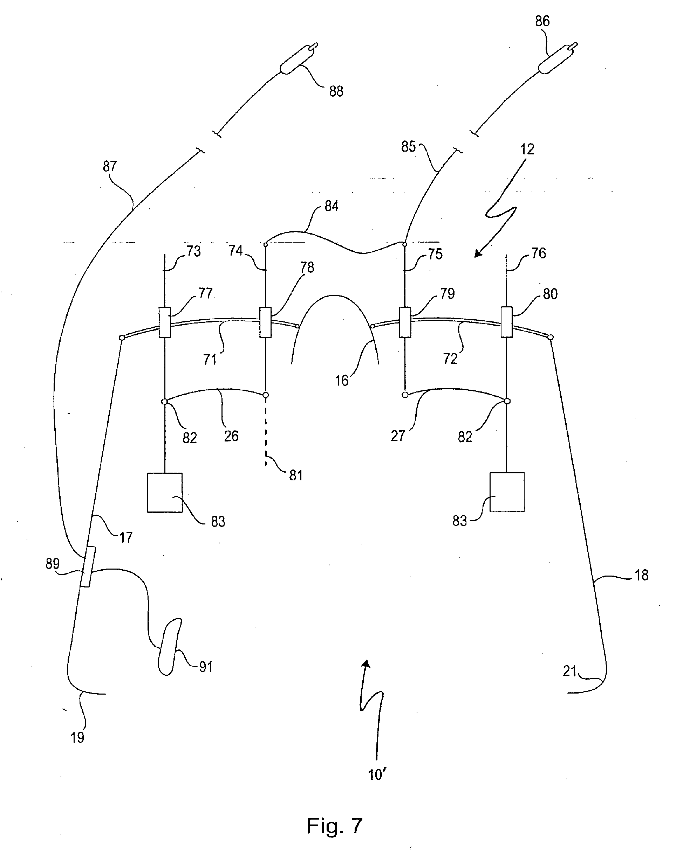

Für die Gegenelektrode ist der Druckknopf 52 vorgesehen, der innerhalb der Gesichtsmaske 45 mit eine Kontaktfläche verbunden ist, die sich beim Aufsetzen der Gesichtsmaske an der Schläfe des Patienten anlegt. Die Gegenelektrode ist in Fig.7 im Zusammenhang mit einem weiteren Ausführungsbeispiel der neuen Vorrichtung 10' gezeigt. Der Druckknopf 52 ist über ein Anschlusskabel 52 mit Stecker 30 an das Stimulationsgerät 36 angeschlossen.

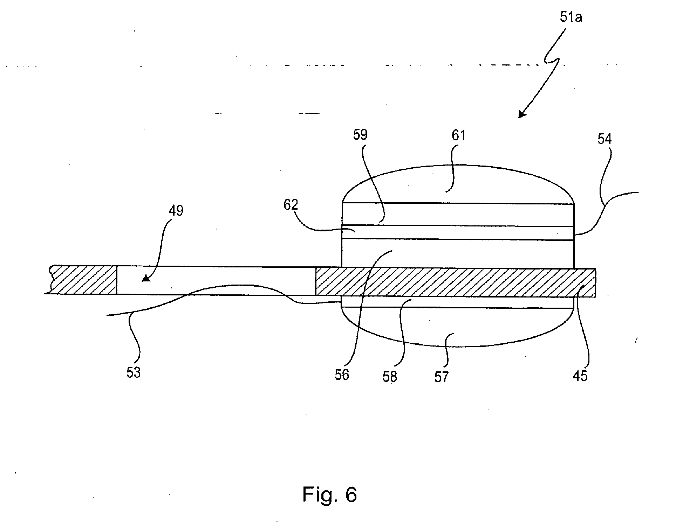

Fig. 6 zeigt einen schematischen Schnitt durch die Gesichtsmaske 45 im Bereich des Augenloches 49, wobei der Druckknopf 51a in Seitenansicht zu erkennen ist.

Der Druckknopf 51a umfasst ein Unterteil 56, das über ein Befestigungsteil 57 an der Gesichtsmaske 45 befestigt ist. Dazu dienen in an sich bekannter Weise Zinken, wie sie in der eingangs erwähnten DE 36 11 115 A1 beschrieben ist. Zwischen dem Befestigungsteil 57 und der Gesichtsmaske 45 ist ein Klemmende 58 der Stimulationselektrode 53 angeordnet und festgelegt.

Der Druckknopf 51 weist ferner ein Oberteil 59 auf, das ebenfalls mit einem Befestigungsteil 61 verbunden ist. Zwischen Unterteil 56 und Oberteil 59 liegt eine Anschlussöse 62 des Anschlusskabels 54, so dass beim Aufklipsen des Oberteiles 59 auf das Unterteil 56 das Anschlusskabel 54 fest mit dem Druckknopf 51a verbunden wird, wobei gleichzeitig für eine elektrisch leitende Verbindung zu der Stimulationselektrode 53 gesorgt wird.

Da der geschlossene Druckknopf 51a fest und unverrückbar an der Gesichtsmaske 45 festgelegt ist, führt ein Zug an dem Anschlusskabel 54 nicht dazu, dass sich die Stimulationselektrode 53 gegenüber der Gesichtsmaske 54 verlagert.

Ferner ist die Gesichtsmaske 45 derart an dem Kopf des Patienten angepasst und dort in geeigneter Weise festgelegt, dass ein Zug an dem Anschlusskabel 54 auch nicht dazu führt, dass sich die Gesichtsmaske 45 gegenüber dem Kopf des Patienten verlagert.

All dies führt dazu, dass die Stimulationselektrode 53 während der Dauer der Stimulation in der einmal eingenommenen Lage verbleibt, so dass für eine sichere und über die Zeit kontinuierliche und gleichbleibende Stimulation gesorgt ist.

Die Stimulationselektrode 53 kann auch oberhalb der Gesichtsmaske 45 an dem Befestigungsteil 57 festgelegt werden, so dass sie sich sozusagen außerhalb der Gesichtsmaske 45 durch die Augenöffnung 49 erstreckt.

In der Fig. 7 ist noch ein weiteres Ausführungsbeispiel der neuen Vorrichtung 10" gezeigt, bei der das Nasenteil 12 anstelle der Rahmen 14 und 15 ggf. leicht gebogene Augenbügel 71, 72 aufweist, die sich zwischen dem Nasenbügel 16 und den Seitenbügeln 17 bzw. 18 erstrecken.

In Fig. 7 sind für übereinstimmende Merkmale dieselben Bezugszeichen wie in Fig. 1 und 3 verwendet, so dass wegen der weiteren Einzelheiten auf die obige Beschreibung verwiesen wird.

Bei der Vorrichtung 10" sind die Elektrodenhalter 22, 23, 24, 25 als Stifte 73, 74, 75, 76 ausgebildet, die aus V2A-Stahl gefertigt sind. Die Stifte 73, 74, 75, 76 sind über Halter 77, 78, 79, 80 an den Augenbügeln 71 bzw. 72 derart befestigt, dass sie in Längsrichtung 81 der Stifte 73, 74, 75, 76 verstellbar und bei Bedarf austauschbar sind.

Aus diese Weise wird die Vorrichtung 10" einmalig von einem Fachmann an die Geometrie des Patientenkopfes derart angepasst, dass die zwischen den Stiften 73 und 74 bzw. 75 und 76 verlaufenden Stimulationselektroden 26 bzw. 27 sich so an das Auge des Patienten anlegen, wie dies oben schon beschrieben wurde.

Da die Stifte 73, 74, 75, 76 starr ausgebildet sind, erfolgt die Anlage der Stimulationselektroden 26, 27 an die Cornea dadurch, dass die Stimulationselektroden als Schlaufen in die augenseitigen, inneren Enden der Stifte 73, 74, 75, 76 eingelegt und jeweils nur mit dem nasenseitigen Stift 74 bzw.75 mechanisch fest und elektrisch leitend verbunden werden. An dem schläfenseitigen Stift 73 bzw. 76 ist die Stimulationselektrode 26 bzw. 27 durch eine Öse 82 zu je einem Gewichtskörper 83 geführt, der eine hinreichende Masse aufweist, um die Stimulationselektroden 26, 27 derart auf Zug zu belasten, dass sie leicht gestrafft werden und sich an die Cornea anlegen.

Die Stimulationselektroden 26, 27 können leicht ausgewechselt werden, sie müssen nur aus den Ösen 82 entnommen und von den nasenseitigen Stiften 74, 75 gelöst werden.

Die nasenseitigen Stifte 74 und 75 sind über ein Kabel 84 elektrisch miteinander verbunden, das zudem eine Verbindung zu einem Anschlusskabel 85 besitzt, das über einen Stecker 86 mit dem Stimulationsgerät 36 verbunden werden kann. Auf diese Weise sind die Stimulationselektroden 26 und 27 sozusagen elektrisch parallel geschaltet. Der zweite Anschluss an das Stimulationsgerät 36 erfolgt über ein Anschlusskabel 87 mit Stecker 88.

Dieses Anschlusskabel 87 ist über einen Halter 89 an dem Seitenbügel 17 festgelegt und führt zu einer Gegenelektrode 91, die an die Schläfe des Patienten angelegt wird und somit den Stromkreis schließt.

Die Gegenelektrode 91 kann auch im Zusammenhang mit der Gesichtsmaske 45 aus Fig. 5 oder einer Vorrichtung 10, 10' aus den Fig., 1 und 3 verwendet werden, wenn dort entweder nur eine Stimulationselektrode 26, 27 oder zwei wie in Fig. 7 elektrisch parallel geschaltete Stimulationselektroden 26, 27 eingesetzt werden.

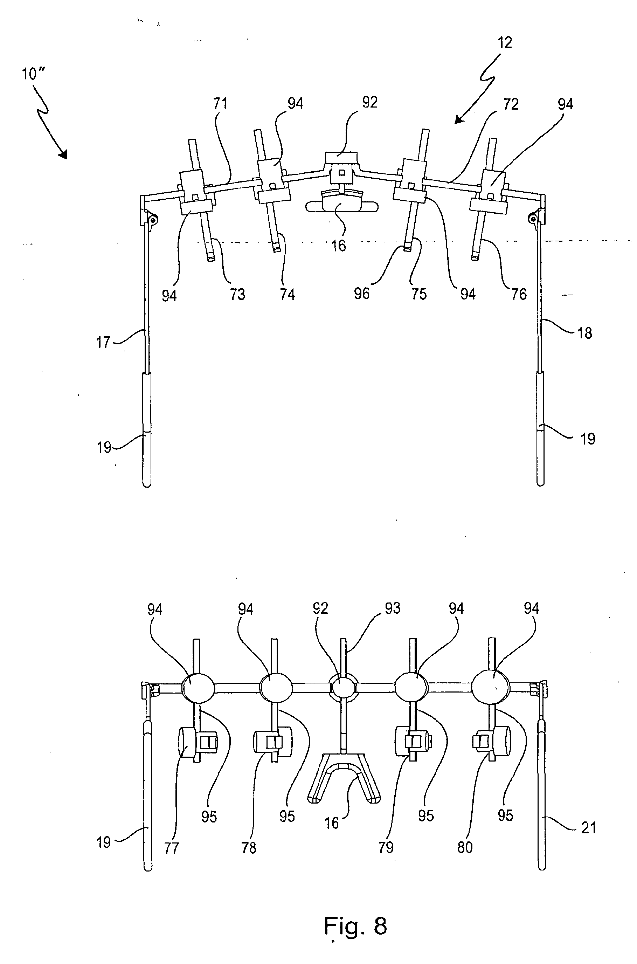

In Fig. 8 ist oben in Draufsicht und unten in Ansicht vom Patienten her eine alternative Ausgestaltung der Vorrichtung 10" gezeigt. Bei dieser Vorrichtung 10'" sind die Augenbügel 71, 72 des Nasenteils 12 gerade ausgebildet und innen unter einem Winkel leicht zum Patienten hin geneigt an einem Nasenhalter 92 befestigt. Außen sind an den Augenbügeln 71, 72 wieder die schon beschriebenen Seitenbügel 17, 18 angelenkt.

An dem Nasenhalter 92 ist über einen Haltestift 93 das Nasenteil 16 befestigt, das somit in Längsrichtung des Haltestiftes 93 gegenüber den Augenbügeln verstellt werden kann, um die Höhe des Nasenteils 12 gegenüber der Nase des Patienten so einstellen zu können, dass die Augenbügel 71, 72 optimal zu dessen Augen ausgerichtet werden können.

Auf jedem Augenbügel 71, 72 sind jeweils zwei Stifthalter 94 angeordnet, die längs der Augenbügel 71, 72 verschiebbar und um die Augenbügel 71, 72 schwenkbar sind. Jeder Stifthalter 94 trägt einen Haltestift 95, der in seiner Längsrichtung verstellbar gehalten wird. Die Haltestifte 95 erstrecken sich etwa parallel zu dem Haltestift 93.

An ihrem freien, unteren Ende tragen die Haltestifte 95 jeweils einen der aus Fig. 7 bekannten Halter 77, 78, 79,80, in denen jeweils einer der als Elektrodenhalter 22, 23, 24, 25 dienenden V2A-Stifte 73, 74, 75, 76 längsverschieblich gehalten ist. An ihrem freien, augennahen Ende sind die Stifte 73, 74, 75, 76 jeweils mit einer noch näher zu beschreibenden Aufnahme 96 für die in Fig. 8 nicht dargestellte Stimulationselektrode versehen.

Augenbügel 71, 72, Haltestifte 95 und Stifte 73, 74, 75, 76 verlaufen etwa orthogonal zueinander, so dass die Aufnahmen 96 in allen drei Raumrichtungen verstellbar und somit an die Gegebenheiten der Kopf-, Gesichts- und Augenform des Patienten problemlos anpassbar sind. Bezogen auf die Gebrauchsstellung der neuen Vorrichtung verlaufen Augenbügel 71, 72 und Stifte 73, 74, 75, 76 etwa horizontal, während die Haltestifte 93 und 95 etwa senkrecht verlaufen.

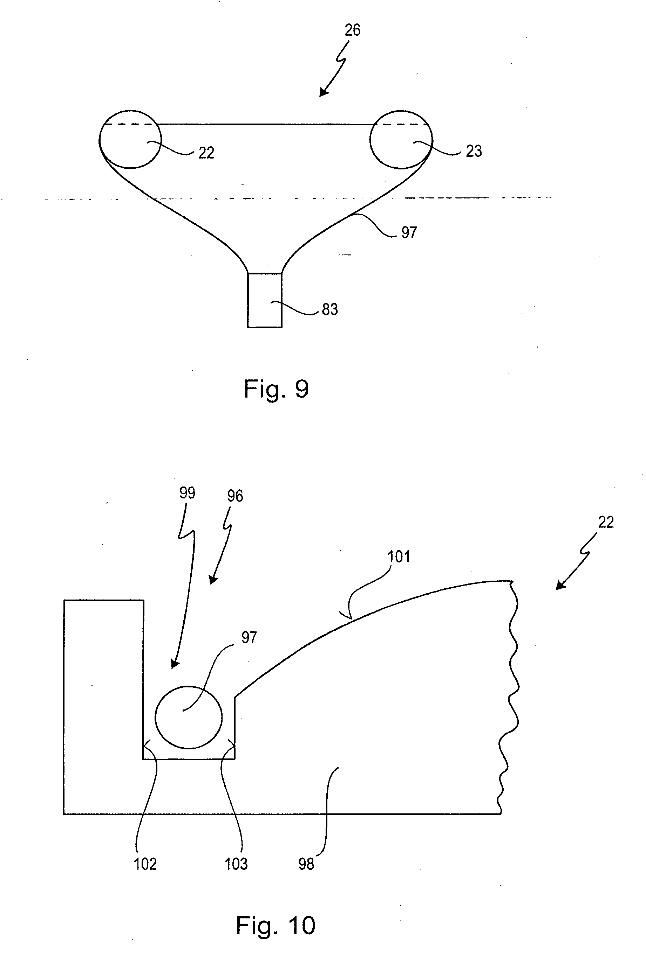

In Fig. 9 ist eine als Schlaufe 97 ausgebildete Stimulationselektrode 26 gezeigt, wie sie bei den Vorrichtungen 10, 10" und 10"' aus den Fig. 1, 3, 7 und 8 eingesetzt werden kann. Diese Schlaufe 97 ist in zwei Elektrodenhalter 22, 23 eingehängt und durch einen mit beiden Enden der Schlaufe 97 verbundenen Gewichtskörper 83 gespannt, der in Gebrauchsstellung unter und zwischen den Elektrodenhaltern 22, 23 hängt.

Als Elektrodenhalter 22, 23 können sowohl die Spiralfedern oder elastischen Stifte gemäß Figuren 1 bis 4 als auch die starren Stifte 73, 74, 75, 76 gemäß Fig. 7 oder 8 verwendet werden. Diese Elektrodenhalter 22, 23 weisen an ihrem freien augennahen Ende 98 die schon erwähnte Aufnahme 34 bzw. 96 auf, die hier eine nach oben offene Nut 99 aufweist, in der die Schlaufe 97 zu liegen kommt.

Um auch stark sehbehinderten oder nahezu blinden Patienten das Einhängen der Schlaufe 97 zu ermöglichen bzw. zu erleichtern, ist an dem Elektrodenhalter 22 eine schräg auf die Nut 99 zu laufende Fläche 101 ausgebildet, wie es in Fig. 10 in stark vergrößerter, nicht maßstabsgetreuer Darstellung gezeigt. Eine auf den Elektrodenhalte 22 aufgelegte Schlaufe 87 rutscht somit automatisch in die Nut 99 hinein, wo sie seitlich durch zwei senkrechte Seitenwände 102, 103 gehalten wird, so dass sie beim Aufsetzen der Vorrichtung 10, 10` 10'" auch dann nicht aus der Nut 99 frei kommt, wenn die Stimulationselektrode sich an die Cornea anlegt und dabei durch den Gewichtskörper 83 gespannt wird.

Damit die Schlaufe 97 elektrisch kontaktiert werden kann, ist zumindest einer der Elektrodenhalter 22, 23 aus einem elektrisch gut leitenden Material gefertigt, wobei der Übergangswiderstand zu der Schlaufe dadurch gering gehalten wird, dass die Schlaufe 97 durch den Gewichtskörper 83 in die Nut 99 hineingezogen wird.

Der betreffende Elektrodenhalter 22, 23 ist dann in schon beschriebener Weise mit einem Kabel verbunden, das dem Anschluss an ein Stimulationsgerät 36 dient, wie dies oben ausführlicher beschrieben wurde.

## Patentansprüche

1. Vorrichtung zur Elektrostimulation des Auges, mit einem brillenartigen Gestell (11), das ein Nasenteil (12) und eine mit dem Nasenteil (12) verbundene Anordnung (17, 18) zum Halten des Gestells (11) an dem Kopf des Patienten aufweist, wobei an dem Nasenteil (12) zumindest eine Stimulationselektrode (26, 27) angeordnet ist,
**dadurch gekennzeichnet, dass** an dem Nasenteil (12) zumindest zwei Elektrodenhalter (22, 23, 24, 25) vorgesehen sind, zwischen denen die Stimulationselektrode (26, 27) in Form eines auswechselbaren, drahtförmigen Stimulationselektrodes (26, 27) eingespannt ist, die so ausgebildet ist, dass sie sich beim Aufsetzen der Vorrichtung an die Cornea (35) anlegt.

2. Vorrichtung nach Anspruch 1, **dadurch gekennzeichnet, dass** die Stimulationselektrode (26, 27) aus einem metallisch beschichteten Kunststofffaden gefertigt ist.

3. Vorrichtung nach Anspruch 1 oder 2, **dadurch gekennzeichnet, dass** die Stimulationselektrode (26, 27) elektrisch leitend mit zumindest einem der Elektrodenhalter (22, 23, 24, 25) verbunden ist.

4. Vorrichtung nach Anspruch 3, **dadurch gekennzeichnet, dass** jeder Elektrodenhalter (22, 23, 24, 25) an seinem freien Ende (47, 98) mit einer Aufnahme (34, 96) für die Stimulationselektrode (26, 27, 97) versehen ist.

5. Vorrichtung nach einem der Ansprüche 1 bis 4, **dadurch gekennzeichnet, dass** mit zumindest einem der Elektrodenhalter (22, 23, 24, 25) ein Kabel (28, 29, 42, 43) verbunden ist.

6. Vorrichtung nach einem der Ansprüche 1 bis 5, **dadurch gekennzeichnet, dass** die Elektrodenhalter (22, 23, 24, 25) mit einem elastisch nachgiebigen, langgestreckten Körper (44) ausgebildet sind.

7. Vorrichtung nach einem der Ansprüche 1 bis 6, **dadurch gekennzeichnet, dass** die Elektrodenhalter (22, 23, 24, 25) als elektrisch leitfähige Stifte (73, 74, 75, 76), vorzugsweise aus V2A-Stahl, ausgebildet sind.

8. Vorrichtung nach Anspruch 7, **dadurch gekennzeichnet, dass** die zumindest eine Stimulationselektrode (26, 27) mit einem der Stifte (74, 75) fest verbunden ist und an dem anderen Stift (73, 76) durch eine Öse (82) geführt und auf Zug belastet ist, vorzugsweise durch einen Gewichtskörper (83).

9. Vorrichtung nach Anspruch 4, **dadurch gekennzeichnet, dass** die Aufnahme (96) eine Nut (99) umfasst, und dass an dem Elektrodenhalter (22, 23, 24, 25) eine schräg auf die Nut (99) zu laufende Fläche (101) vorgesehen ist.

10. Vorrichtung nach Anspruch 9, **dadurch gekennzeichnet, dass** die zumindest eine Stimulationselektrode (26, 27) als Schlaufe (97) ausgebildet ist, die mit einem Gewichtskörper (83) verbunden und in die Nuten (99) von zwei Elektrodenhaltern (2, 23) eingehängt ist.

11. Vorrichtung nach einem der Ansprüche 1 bis 10, **dadurch gekennzeichnet, dass** jeder Elektrodenhalter (22, 23, 24, 25) eine Halterung (46, 77, 78, 79, 80) aufweist, über die er verstellbar und austauschbar an dem Nasenteil (12) angeordnet ist.

12. Vorrichtung nach einem der Ansprüche 1 bis 11, **dadurch gekennzeichnet, dass** das Nasenteil (12) einen Nasenbügel (16) und zumindest einen Rahmen (14, 15), oder Augenbügel (71, 72) aufweist, an dem die beiden Elektrodenhalter (22, 23, 24, 25) angeordnet sind, wobei der Nasenbügel (16) gegenüber dem Rahmen (14) oder den Augenbügeln (71, 72) verstellbar angeordnet ist, und der Nasenbügel (16) über einen Haltestift (93) an einem Nasenhalter (92) verstellbar angeordnet ist, an dem zwei Augenbügel (71, 72) festgelegt sind, von denen zumindest einer mit zwei Stifthaltern (94) versehen sind, die verschiebbar auf dem Augenbügel (71, 72) sitzen und von denen jeder einen höhenverstellbaren Haltestift (95) trägt, an dem jeweils ein in seiner Längsrichtung verstellbarer Stift (73, 74, 75, 76) als Elektrodenhalter (22, 23, 24, 25) vorgesehen ist.

13. Vorrichtung nach einem der Ansprüche 1 bis 12, **dadurch gekennzeichnet, dass** eine Gegenelektrode (91) für die zumindest eine Stimulationselektrode (26, 27) vorgesehen ist.

14. Vorrichtung nach Anspruch 13, **dadurch gekennzeichnet, dass** zwei Stimulationselektroden (26, 27) vorgesehen und elektrisch parallel geschaltet sind.

15. Stimulationselektrode zu Verwendung mit einer Vorrichtung nach einem der Ansprüche 1 bis 14, **dadurch gekennzeichnet, dass** sie als Schlaufe (97) aus einem metallisch beschichteten Kunststofffaden ausgebildet und mit einem Gewichtskörper (83) verbunden ist.

## Claims

1. A device for electrostimulation of the eye, comprising a spectacles-like supporting frame (11) which comprises a nose part (12) and an arrangement (17, 18) that is connected to the nose part (12) for holding the supporting frame (11) on the head of the patient, wherein at least one stimulation electrode (26, 27) is arranged on the nose part (12),
**characterized in that** at least two electrode holders (22, 23, 24, 25) are provided on the nose part (12), between which electrode holders said stimulation electrode (26, 27) is clamped in form of an interchangeable, wire-shaped stimulation electrode (26, 27), said stimulation electrode being configured such that it comes to rest against the cornea (35) when the device is put on.

2. The device as claimed in claim 1, **characterized in that** the stimulation electrode (26, 27) is manufactured from a metallically coated polymer thread.

3. The device as claimed in claim 1 or 2, **characterized in that** the stimulation electrode (26, 27) is connected to at least one of the electrode holders (22, 23, 24, 25) in an electrically conductive fashion.

4. The device as claimed in claim 3, **characterized in that**, at its free end (47, 98), each electrode holder (22, 23, 24, 25) is provided with a receptacle (34, 96) for the stimulation electrode (26, 27, 97).

5. The device as claimed in anyone of claims 1 to 4, **characterized in that** a cable (28, 29, 42, 43) is connected to at least one of the electrode holders (22, 23, 24, 25).

6. The device as claimed in anyone of claims 1 to 5, **characterized in that** the electrode holders (22, 23, 24, 25) are embodied with an elastically resilient, elongate body (44).

7. The device as claimed in anyone of claims 1 to 6, **characterized in that** the electrode holders (22, 23, 24, 25) are embodied as electrically conductive pins (73, 74, 75, 76), preferably made of V2A stainless steel.

8. The device as claimed in claim 7, **characterized in that** the at least one stimulation electrode (26, 27) is fixedly connected to one of the pins (74, 75) and, at the other pin (73, 76), it is guided through an ear (82) and subjected to tension, preferably by a weight (83).

9. The device as claimed in claim 4, **characterized in that** the receptacle (96) comprises a groove (99), and that an area (101) running obliquely toward the groove (99) is provided on the electrode holder (22, 23, 24, 25).

10. The device as claimed in claim 9, **characterized in that** the at least one stimulation electrode (26, 27) is embodied as a loop (97), which loop is connected to a weight (83) and hooked into the grooves (99) of two electrode holders (2, 23).

11. The device as claimed in anyone of claims 1 to 10, **characterized in that** each electrode holder (22, 23, 24, 25) comprises a mount (46, 77, 78, 79, 80), by means of which mount it is arranged on the nose part (12) in an adjustable and interchangeable fashion.

12. The device as claimed in anyone of claims 1 to 11, **characterized in that** the nose part (12) comprises a nose bracket (16) and at least one frame (14, 15) or eye bracket (71, 72), on which the two electrode holders (22, 23, 24, 25) are arranged, the nose bracket (16) being arranged in adjustable fashion with respect to the frame (14) or eye bracket (71, 72), and **in that** the nose bracket (16) is arranged in adjustable fashion on a nose holder (92) via a holding pin (93), on which nose holder two eye brackets (71, 72) are secured, at least one of which is provided with two pin holders (94) which sit in displaceable fashion on the eye bracket (71, 72) and each of which carries a height-adjustable holding pin (95) on which pin holder respectively one pin (73, 74, 75, 76) is provided, which can be adjusted in its longitudinal direction and serves as an electrode holder (22, 23, 24, 25).

13. The device as claimed in anyone of claims 1 to 12, **characterized in that** a counter electrode (91) is provided for the at least one stimulation electrode (26, 27).

14. The device as claimed in claim 13, **characterized in that** two stimulation electrodes (26, 27) are provided and electrically connected in parallel.

15. A stimulation electrode for use with a device as claimed in anyone of claims 1 to 14, **characterized in that** it is embodied as a loop (97) made of a metallically coated polymer thread and connected to a weight (83).

## Revendications

1. Dispositif servant à l'électrostimulation de l'oeil, comprenant une monture (11) de type lunette, qui présente une partie de nez (12) et un ensemble (17, 18) relié à la partie de nez (12) servant à maintenir la monture (11) au niveau de la tête du patient, sachant qu'au moins une électrode de stimulation (26, 27) est disposée au niveau de la partie de nez (12),
**caractérisé en ce qu'**au moins deux supports d'électrode (22, 23, 24, 25) sont prévus au niveau de la partie de nez (12), entre lesquels l'électrode de stimulation (26, 27) se présentant sous la forme d'une électrode de stimulation (26, 27) remplaçable, en forme de fil métallique est serrée, laquelle est réalisée de telle sorte qu'elle se place, lors de la mise en place du dispositif, contre la cornée (35).

2. Dispositif selon la revendication 1, **caractérisé en ce que** l'électrode de stimulation (26, 27) est fabriquée à partir d'un fil en plastique revêtu d'une couche en métal.

3. Dispositif selon la revendication 1 ou 2, **caractérisé en ce que** l'électrode de stimulation (26, 27) est reliée électriquement à au moins un des supports d'électrode (22, 23, 24, 25).

4. Dispositif selon la revendication 3, **caractérisé en ce que** chaque support d'électrode (22, 23, 24, 25) est pourvu, au niveau de son extrémité (47, 98) libre, d'un logement (34, 96) destiné à l'électrode de stimulation (26, 27, 97).

5. Dispositif selon l'une quelconque des revendications 1 à 4, **caractérisé en ce qu'**un câble (28, 29, 42, 43) est relié à au moins l'un des supports d'électrode (22, 23, 24, 25).

6. Dispositif selon l'une quelconque des revendications 1 à 5, **caractérisé en ce que** les supports d'électrode (22, 23, 24, 25) sont réalisés avec un corps allongé (44) souple élastiquement.

7. Dispositif selon l'une quelconque des revendications 1 à 6, **caractérisé en ce que** les supports d'électrode (22, 23, 24, 25) sont réalisés sous la forme de broches (73, 74, 75, 76) électroconductrices, de préférence en acier V2A.

8. Dispositif selon la revendication 7, **caractérisé en ce que** la au moins une électrode de stimulation (26, 27) est reliée de manière solidaire à l'une des broches (74, 75) et est guidée, au niveau de l'autre broche (73, 76), à travers un oeillet (82) et est soumise à une traction, de préférence par un contrepoids (83).

9. Dispositif selon la revendication 4, **caractérisé en ce que** le logement (96) comprend une rainure (99), et **en ce qu'**une surface (101) s'étendant de manière oblique en direction de la rainure (99) est prévue au niveau du support d'électrode (22, 23, 24, 25).

10. Dispositif selon la revendication 9, **caractérisé en ce que** la au moins une électrode de stimulation (26, 27) est réalisée sous la forme d'une boucle (97), qui est reliée à un contrepoids (83) et est accrochée dans les rainures (99) par deux supports d'électrode (2, 23).

11. Dispositif selon l'une quelconque des revendications 1 à 10, **caractérisé en ce que** chaque support d'électrode (22, 23, 24, 25) présente une fixation (46, 77, 78, 79, 80), par l'intermédiaire de laquelle chaque support d'électrode est disposé de manière à pouvoir être ajusté et remplacé au niveau de la partie de nez (12).

12. Dispositif selon l'une quelconque des revendications 1 à 11, **caractérisé en ce que** la partie de nez (12) présente un pontet de nez (16) et au moins un cadre (14, 15) ou pontet d'oeil (71, 72), au niveau duquel les deux supports d'électrode (22, 23, 24, 25) sont disposés, sachant que le pontet de nez (16) est disposé de manière à pouvoir être ajusté par rapport au cadre (14) ou aux pontets d'oeil (71, 72), et le pontet de nez (16) est disposé de manière à pouvoir être ajusté au niveau d'un support de nez (92) par l'intermédiaire d'une broche de maintien (93), au niveau duquel deux pontets d'oeil (71, 72) sont fixés, dont au moins l'un est pourvu de deux supports de broche (94), qui reposent de manière à pouvoir être coulissés sur le pontet d'oeil (71, 72) et dont chacun supporte une broche de maintien (95) pouvant être ajustée en hauteur, au niveau de laquelle est prévue respectivement une broche (73, 74, 75, 76) pouvant être ajustée dans son sens longitudinal sous la forme d'un support d'électrode (22, 23, 24, 25).

13. Dispositif selon l'une quelconque des revendications 1 à 12, **caractérisé en ce qu'**une contre-électrode (91) est prévue pour la au moins une électrode de stimulation (26, 27).

14. Dispositif selon la revendication 13, **caractérisé en ce que** deux électrodes de stimulation (26, 27) sont prévues et sont montées électriquement en parallèle.

15. Electrode de stimulation destinée à être utilisée avec un dispositif selon l'une quelconque des revendications 1 à 14, **caractérisée en ce qu'**elle est réalisée sous la forme d'une boucle (97) composée d'un fil en plastique revêtu d'une couche en métal et est reliée à un contrepoids (83).
